# EUROPEAN PATENT APPLICATION

(11) **EP 1 978 375 A1**
(43) Date of publication of application: **08.10.2008**
(21) Application number: 08005159.2
(22) Date of filing: 19.03.2008
(51) Int. Cl.: G01S 15/89, A61B 8/00, A61B 8/12

(54) **Ultrasound observation system and ultrasound observation method therefor**

(30) Priority: 04.04.2007 JP 2007098852
(71) Applicant: Olympus Medical Systems Corporation, Tokyo 151-0072 (JP)
(72) Inventor: Okuno, Yoshiyuki, Tokyo 151-0072 (JP)
(74) Representative: von Hellfeld, Axel

(57) **Abstract**

An ultrasound observation apparatus according to the present invention has a transmitting and receiving section (30), an ultrasound signal processing section (31), a CPU (22), a main memory (33), two compact flash memories(TM) (34,35), a vide signal outputting section (36), an output image generating section (7), a device I/F section (38), a keyboard I/F section (37), and a timing controller (8). The ultrasound observation apparatus configured in this way generates an ultrasound observation image and changes the frequency of updating the displayed ultrasound observation image (frame rate) according to the display range of the ultrasound observation image, thereby improving frame rate.

## Description

### BACKGROUND OF THE INVENTION

### 1. Field of the Invention

The present invention relates to an ultrasound observation system and an ultrasound observation method therefor. More specifically, it relates to an ultrasound observation system for observing an affected area by ultrasonotomography and an ultrasound observation method therefor.

### 2. Description of the Related Art

As described in Japanese Patent Application Laid-Open No. 11-33029, for example, in recent years, an ultrasound diagnostic apparatus that emits an ultrasound into a living body and obtains an ultrasound image from the received reflected ultrasound has become widely used for observation or diagnosis of an affected part or the like or, if necessary, for tissue sampling with a puncture needle, because the ultrasound diagnosis apparatus does not need dissection to acquire information about the inside of the living body.

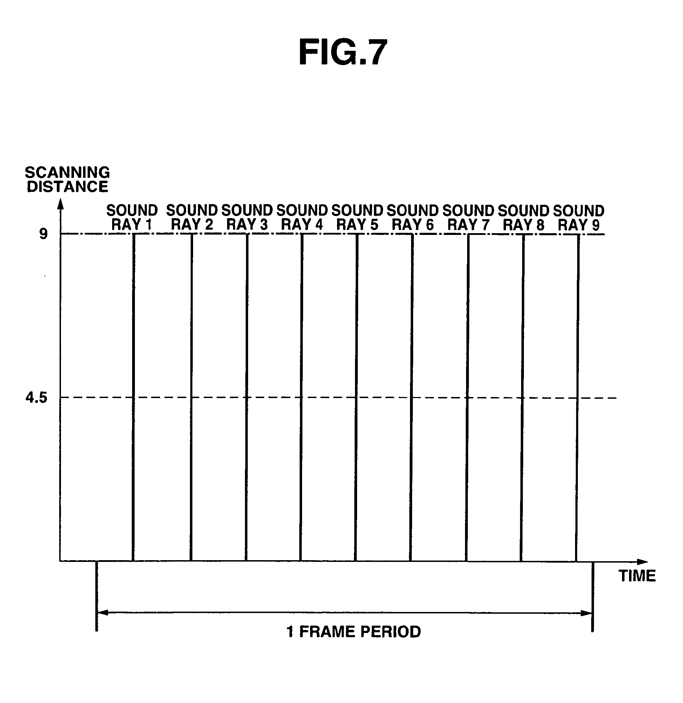

For example, a conventional ultrasound observation apparatus detects and acquires sound ray data in one frame period at timings shown in Fig. 7 and generates and displays an ultrasound observation image.

However, the conventional ultrasound observation apparatus displays the ultrasound observation image (a B mode tomographic image, for example) on a monitor at a fixed frame rate. More specifically, as shown in Fig. 7, the number of times of acquisition of sound ray data in one frame is fixed independently of the ultrasound scanning distance, and therefore, the display frame rate cannot be increased. Thus, the operator can only see the ultrasound image updated at long intervals (at a low frame rate), so that the observation cannot be carried out appropriately.

### SUMMARY OF THE INVENTION

The present invention has been devised in view of such circumstances, and an object of the present invention is to provide an ultrasound observation system that can generate an ultrasound observation image and change the frequency of updating the displayed ultrasound observation image (frame rate) according to the display range of the ultrasound observation image thereby improving frame rate, and an ultrasound observation method for the ultrasound observation system.

An ultrasound observation system according to the present invention comprises:
an ultrasound probe having an ultrasound transducer that transmits and receives an ultrasound; and
an ultrasound observation apparatus that has an ultrasound driving section that drives the ultrasound transducer to make the ultrasound transducer transmit the ultrasound and receives an ultrasound echo signal and an ultrasound observation image data generating section that generates ultrasound observation image data including at least an ultrasound image based on the ultrasound echo signal from the ultrasound driving section, and
the ultrasound observation apparatus further has:
   a display range specifying section that specifies a desired specified range within a display range of a display section for displaying the ultrasound image;
   an ultrasound scanning range setting section that sets a scanning range of the ultrasound driving section based on the specified range; and
   an ultrasound transmission timing controlling section that controls the timing of transmission of the ultrasound of the ultrasound driving section based on the specified range.

Other features and advantages of the present invention will be apparent from the detailed description of the preferred embodiment(s) below.

### BRIEF DESCRIPTION OF THE DRAWINGS

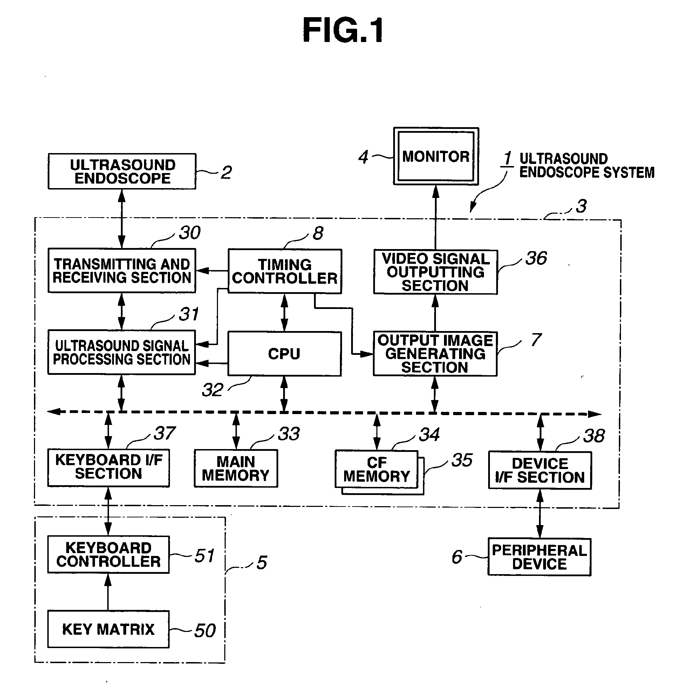

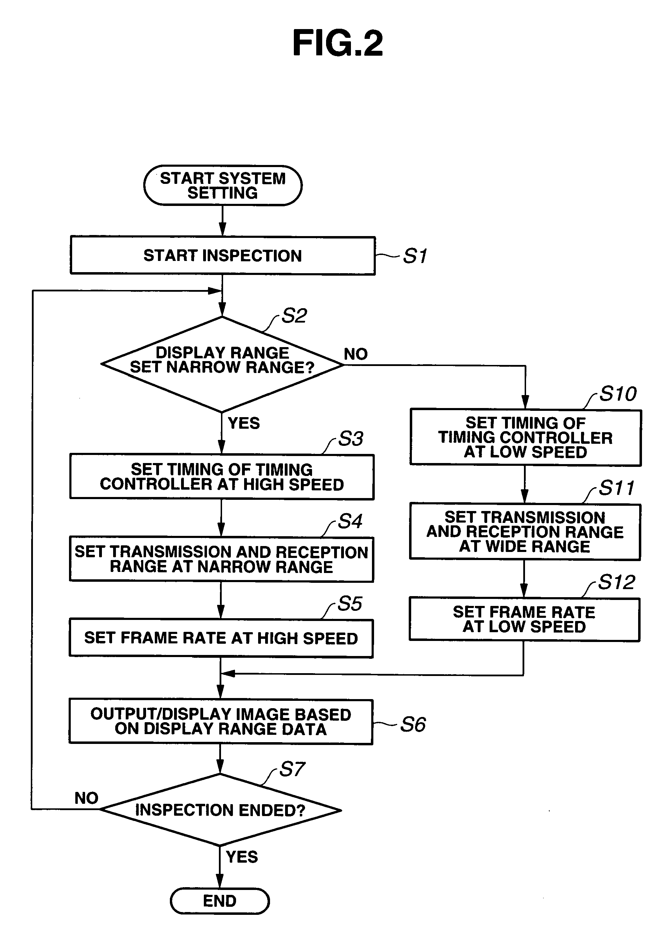

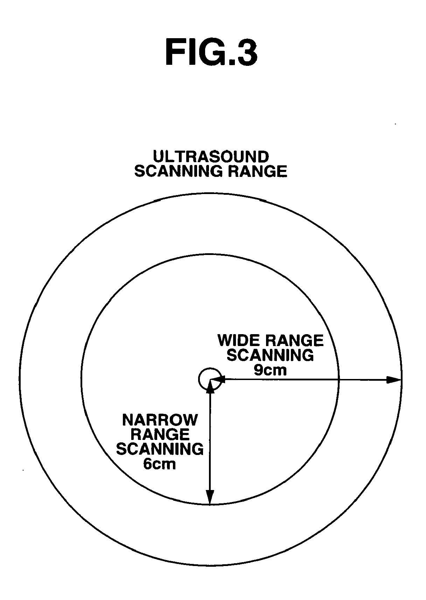

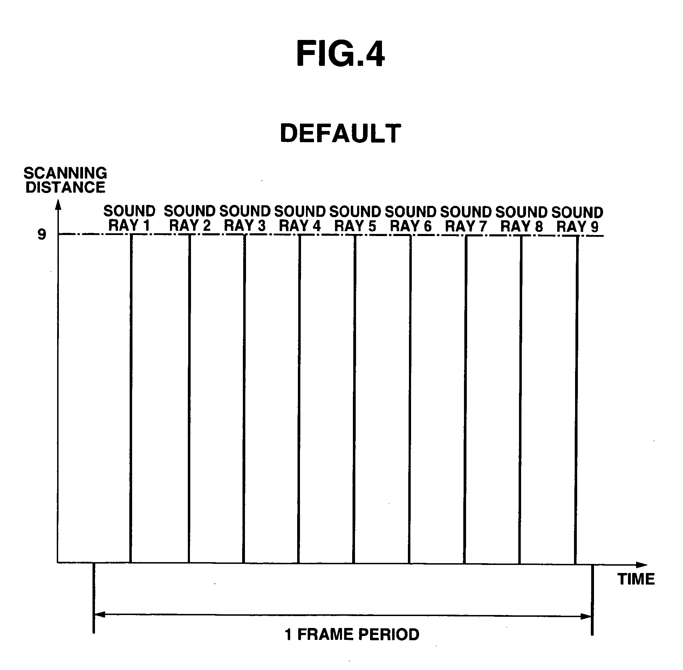

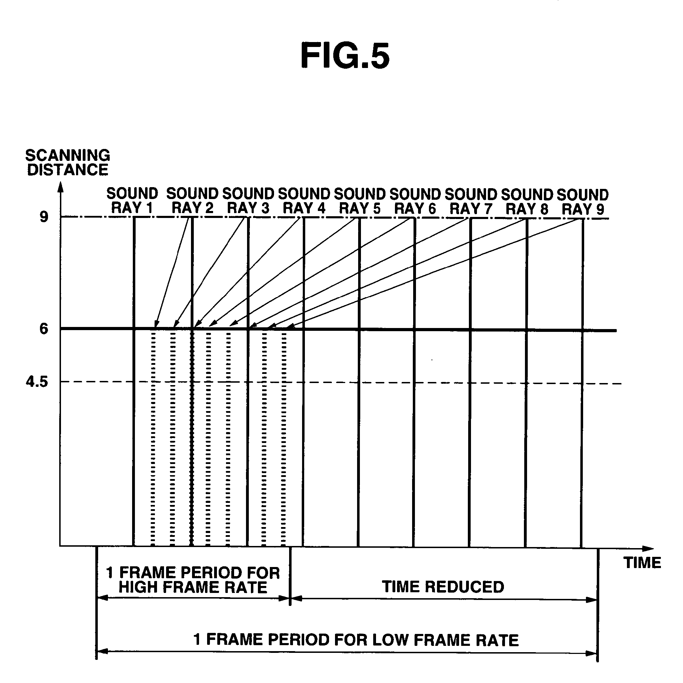

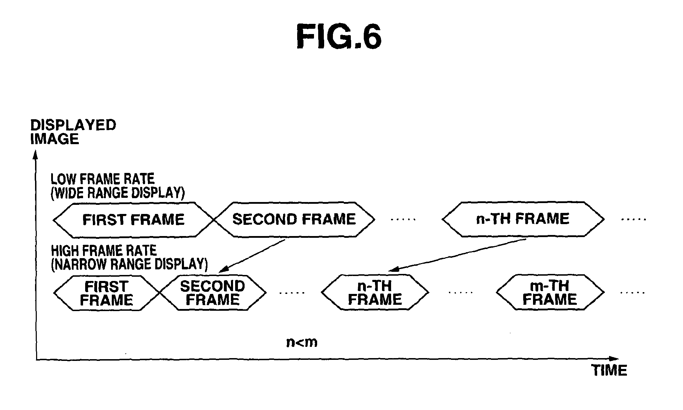

Figs. 1 to 6 relate to an embodiment 1 of the present invention, Fig. 1 is a configuration diagram showing a configuration of an ultrasound endoscope system, Fig. 2 is a flowchart for illustrating an operation of the ultrasound endoscope system shown in Fig. 1, Fig. 3 is a first diagram for illustrating the process shown in Fig. 2, Fig. 4 is a second diagram for illustrating the process shown in Fig. 2, Fig. 5 is a third diagram for illustrating the process shown in Fig. 2, and Fig. 6 is a fourth diagram for illustrating the process shown in Fig. 2.

Fig. 7 is a diagram for illustrating an operation of a conventional ultrasound endoscope system.

### DETAILED DESCRIPTION OF THE PREFERRED EMBODIMENTS

### Embodiment 1

As shown in Fig. 1, an ultrasound endoscope system 1, which is an ultrasound observation system, comprises an ultrasound endoscope 2 that serves as an ultrasound probe that is inserted in a body cavity and transmits and receives an ultrasound signal, an ultrasound observation apparatus 3 that drives an ultrasound element (not shown) of the ultrasound endoscope 2 and processes an ultrasound echo signal to generate an ultrasound image, and a keyboard 5 serving as a display range controlling section for inputting various command signals to the ultrasound observation apparatus 3.

The ultrasound observation apparatus 3 comprises a transmitting and receiving section 30, an ultrasound signal processing section 31 that serves as an ultrasound observation image data generating section, a CPU 32, a main memory 33, two compact flash(TM) memories (CF memories) 34 and 35, a video signal outputting section 36, an output image generating section 7, a device I/F section 38, a keyboard I/F section 37, and a timing controller 8 that serves as an ultrasound scan range setting section and an ultrasound transmission timing controlling section.

The transmitting and receiving section 30 transmits a drive signal to the ultrasound element of the ultrasound endoscope 2 in response to a timing signal from the timing controller 8 and receives an ultrasound echo signal from the ultrasound element.

The ultrasound signal processing section 31 generates the drive signal in response to a timing signal from the timing controller 8 and generates various ultrasound images (a B mode tomographic image, for example) from the ultrasound echo signal.

For example, the ultrasound endoscope 2 is a mechanical scanning ultrasound endoscope having a mechanical scanning type ultrasound transducer disclosed in Japanese Patent Application Laid-Open No. 2000-279415, and the ultrasound signal processing section 31 generates various ultrasound images from the ultrasound echo signal by a signal processing disclosed in Japanese Patent Application Laid-Open No. 2000-279415. Thus, the ultrasound endoscope 2 and the ultrasound signal processing section 31 are well known in the art, and therefore, descriptions thereof will be omitted.

However, the ultrasound endoscope 2 is not limited to the mechanical scanning type ultrasound endoscope. For example, the ultrasound endoscope 2 can be an electronic scanning type ultrasound endoscope disclosed in Japanese Patent Application Laid-Open No. 7-163561 (paragraph [0011]). Details thereof are well known in the art, and therefore, descriptions thereof will be omitted.

The CPU 32 is a controlling section that controls the whole of the ultrasound observation apparatus 3 and operates according to a system program stored in the main memory 33.

Under the control of the CPU 32, the timing controller 8 controls the transmission and reception timing of the transmitting and receiving section 30, the processing timing of the ultrasound signal processing section 31, and the image generation timing of the output image generating section 7.

The CF memory 34 is a storage section that stores an application program activated by the CPU 32, and the CF memory 35 is a storage section that stores the ultrasound image generated by the ultrasound signal processing section 31.

The output image generating section 7 converts the size of the ultrasound image generated by the ultrasound signal processing section 31 to a display size corresponding to the display range specified via the keyboard 5 in response to a timing signal from the timing controller 8 and outputs the converted ultrasound image to the video signal outputting section 36.

The video signal outputting section 36 outputs the ultrasound image of the display size corresponding to the display range specified via the keyboard 5 received from the output image generating section 7 to an observing monitor 4.

The device I/F section 38 is an interface for transmitting data to and receiving data with various peripheral devices 6 connected to the ultrasound observation apparatus 3, such as a printer (video printer) and an information recording device (image file device).

The keyboard I/F section 37 is an interface with the keyboard 5, which comprises a key matrix 50 and a keyboard controller 51.

The key matrix 50 comprises a group of switches including a plurality of switches for inputting data, including display range button (not shown) used for specifying the display range.

The keyboard controller 51 is a controlling section that manages the operational state of the switches of the key matrix 50 and controls the whole of the keyboard 5.

An operation of the ultrasound endoscope system 1 according to the present embodiment configured as described above will be described. Once the ultrasound endoscope 2 and the keyboard 5 are connected to the ultrasound observation apparatus 3, and inspection is started in step S 1 as shown in Fig. 2, in step S2, the CPU 32 determines whether the display range button (not shown) of the keyboard 5 is set at a narrow range (mode) or not.

If it is determined that the display range is set at the narrow range (mode), in step S3, the CPU 32 sets a timing of the timing controller 8 at a high speed (mode).

Then, in step S4, the CPU 32 sets a sampling timing at a high speed (mode) and sets a transmission and reception range of the transmitting and receiving section 30 at a narrow range (a circular range having a radius of 6 cm, for example), as shown in Fig. 3.

Then, in step S5, the CPU 32 sets a default frame rate of the ultrasound signal processing section 31 shown in Fig. 4 at a high frame rate (for narrow range display) as shown in Figs. 5 and 6.

The CPU 32 carries out the narrowing down of the transmission and reception range and the raise of the frame rate described above according to a timing signal from the timing controller 8.

Then, in step S6, the CPU 32 controls the output image generating section 7 to read display range data in response to a timing signal from the timing controller 8 and output the display range data to the video signal outputting section 36, thereby outputting and displaying an ultrasound image on the observing monitor 4.

Then, in step S7, the CPU 32 repeats the steps S2 to S7 until the end of the inspection is detected.

On the other hand, if it is determined in step S2 that the display range is set at a wide range (mode), in step S10, the CPU 32 sets the timing of the timing controller 8 at a low speed (mode).

Then, in step S11, the CPU 32 sets the sampling timing at a low speed (mode) and sets the transmission and reception range of the transmitting and receiving section 30 at a wide range (a circular range having a radius of 9 cm, for example), as shown in Fig. 3.

Then, in step S12, the CPU 32 sets the frame rate of the ultrasound signal processing section 31 at a low frame rate (for wide range display) as shown in Figs. 5 and 6, and the process proceeds to step S6.

The CPU 32 carries out the widening of the transmission and reception range and the lowering of the frame rate described above according to a timing signal from the timing controller 8.

As described above, according to the present embodiment, the scanning range and the frame rate are automatically set according to the setting of the display range button of the keyboard 5, and the resulting ultrasound image is output to and displayed on the observing monitor 4. Therefore, the operator can advantageously observe the ultrasound image of a desired region at a frame rate free from discomfort.

That is, according to the present embodiment, the ultrasound observation image can be generated and the frequency of updating the displayed image (frame rate) can be changed according to the display range of the ultrasound observation image, thereby improving frame rate.

It is apparent that a wide variety of different embodiments are possible without departing from the spirit and scope of the present invention. The present invention is not limited to any particular embodiment but is limited only by the accompanying claims.

## Claims

1. An ultrasound observation system, comprising:
an ultrasound probe having an ultrasound transducer that transmits and receives an ultrasound; and
an ultrasound observation apparatus that has an ultrasound driving section that drives the ultrasound transducer to make the ultrasound transducer transmit the ultrasound and receives an ultrasound echo signal and an ultrasound observation image data generating section that generates ultrasound observation image data including at least an ultrasound image based on the ultrasound echo signal from the ultrasound driving section;
wherein the ultrasound observation apparatus further has:
a display range specifying section that specifies a desired specified range within a display range of a display section for displaying the ultrasound image;
an ultrasound scanning range setting section that sets a scanning range of the ultrasound driving section based on the specified range; and
an ultrasound transmission timing controlling section that controls the timing of transmission of the ultrasound of the ultrasound driving section based on the specified range.

2. The ultrasound observation system according to claim 1, wherein the display range specifying section comprises a keyboard, and the keyboard has a range button for specifying the specified range.

3. The ultrasound observation system according to claim 1 or 2, further comprising:
a controlling section that controls the ultrasound scanning range setting section and the ultrasound scanning range setting section based on the specified range.

4. The ultrasound observation system according to claim 3, wherein the controlling section outputs a control signal for setting a frame rate of the ultrasound observation image data to the ultrasound transmission timing controlling section based on the specified range.

5. The ultrasound observation system according to any one of claims 2 to 4, wherein the range button comprises a plurality of button keys for specifying whether the specified range is a wide range or a narrow range.

6. The ultrasound observation system according to any one of claims 1 to 5, wherein the ultrasound probe is an ultrasound endoscope capable of being inserted in a body cavity.

7. An ultrasound observation method for an ultrasound observation system that has an ultrasound driving section that drives an ultrasound transducer of an ultrasound probe that transmits and receives an ultrasound to make the ultrasound transducer transmit the ultrasound and receives an ultrasound echo signal and an ultrasound observation image data generating section that generates ultrasound observation image data including at least an ultrasound image based on the ultrasound echo signal from the ultrasound driving section, the method comprising:
a display range specifying step of specifying a desired specified range within a display range of a display section for displaying the ultrasound image;
an ultrasound scanning range setting step of setting a scanning range of the ultrasound driving section based on the specified range; and
an ultrasound transmission timing controlling step of controlling the timing of transmission of the ultrasound of the ultrasound driving section based on the specified range.

8. The ultrasound observation method for an ultrasound observation system according to claim 7, further comprising:
a step of outputting a control signal, which is used in the ultrasound transmission timing controlling step, for setting a frame rate of the ultrasound observation image data based on the specified range.

9. The ultrasound observation method for an ultrasound observation system according to claim 8, wherein the control signal sets the frame rate at least based on whether the specified range is a wide range or a narrow range.

10. The ultrasound observation method for an ultrasound observation system according to any one of claims 7 to 9, wherein the ultrasound probe is an ultrasound endoscope capable of being inserted in a body cavity.
